# EUROPEAN PATENT APPLICATION

(11) **EP 2 826 435 A1**
(43) Date of publication of application: **21.01.2015**
(21) Application number: 14180048.2
(22) Date of filing: 21.01.2004
(51) Int. Cl.: A61B 18/14

(54) **Surgical perforation device with ECG monitoring, pressure monitoring, curve and staining abilities.**

(30) Priority: 21.01.2003 US 347366; 19.09.2003 US 666301; 19.09.2003 US 666288
(62) Divisional of application: 11166255.7
(71) Applicant: Baylis Medical Company Inc., Montreal, Quebec H4T 1A1 (CA)
(72) Inventor: Visram, Naheed, Markham, Ontario L3R 9E9 (CA); Shah, Krishan, Mississauga, Ontario L5M 2C7 (CA); Hartley, Amanda April, Brampton, Ontario L7A 1P1 (CA); Baylis, Frank, Beaconsfield, Québec H9W 1R8 (CA)
(74) Representative: Turner, Craig Robert

(57) **Abstract**

A device for creating a controlled transseptal perforation, the device comprises:
- a tubular elongate member defining a proximal region and a distal region;
- at least one electrode associated with the distal region, the at least one electrode being capable of creating the controlled transseptal perforation in body tissue through the application of radiofrequency energy; and
- a means for determining a position of the device inside a patient's body.

## Description

### TECHNICAL FIELD

The invention relates to a surgical perforation method and a surgical device with, ECG monitoring, pressure monitoring and staining ability for creating a perforation in a patient material while minimizing the risk of inadvertent injury to other patient structures. More specifically, the invention relates to a device and method for creating a controlled perforation in the atrial septum while using ECG to locate the fossa ovalis region on the atrial septum, staining the fossa ovalis region, monitoring blood pressure and delivering a dilator and guiding sheath to the left atrium through the perforation over the surgical device while minimizing the risk of inadvertent injury to the left atrium or other areas of the heart.

### BACKGROUND OF THE ART

Electrosurgical devices perforate or cut tissues when radio frequency (RF) electrical energy rapidly increases tissue temperature to the extent that the intracellular fluid becomes converted to steam, inducing cell lysis as a result of elevated pressure within the cell. The radio frequency range lies between 10kHz and 300MHz, but electrosurgical devices usually operate at a frequency between 400 kHz and 550 kHz. This technology can be used to create perforations in different types of tissue, such as heart tissue, vascular occlusions, and others. Commonly, RF devices are described for use in perforating vascular occlusions.

A device to dilate and/or lance blood vessels that are morbidly contracted or clogged is described in European Patent Application Publication Number EP 0315730, of Osypka, published May 15, 1989. The device described therein uses RF energy in either bipolar or monopolar application modes to open blood vessels by means of heat. Other devices intended to use RF energy to pass through occluded vessels have also been described (U.S. Patent No. 5364393, of Auth et al., issued November 15, 1994, WO 93/20747, publication of PCT Patent Application No. PCT/US93/03759, of Rosar, published October 28, 1993, U.S. Patent No. 5098431, of Rydell, issued March 24, 1992, and U.S. Patent No. 4682596 of Bales et al., issued July 28, 1987). U.S. Patent No. 6293945 B1, of Parins et al., issued September 25, 2001 describes an electrosurgical instrument with suction capability. This device has three functions at the tip including cutting, coagulating, and suction. None of these devices however incorporate a means for verifying the location of the device within the body.

One means for verifying location is described in U.S. Patent No. 4936281, of Stasz, issued June 26, 1990, which describes an ultrasonically enhanced RF catheter used for cutting. An ultrasonic transducer connected to an electronics module receives echo signals, enabling Doppler flow readings and ultrasound imaging of the vessel.

Having reliable information about the location of electrosurgical devices within a body is an important aid to performing a successful procedure. It is often valuable to have more than one source of this information because every imaging technique has limitations, and using only one method can lead to erroneous information. A number of different tools and techniques have been used to assist the physician in locating devices within a heart. These include the use of fluoroscopy, cardiac pressure monitoring, transesophageal echocardiography, intracardiac echocardiography and the use of other devices in the heart to identify certain landmarks.

Relative blood pressure measurements can be a useful tool to verify the position of a device in a body. Different locations in the body are known to have characteristic blood pressure ranges. Knowing the blood pressure at the tip of a perforation device is a useful tool to determine the location of the device, particularly in instances where imaging techniques provide inconclusive information. A device that is used for measuring pressure in coronary arteries is described in U.S. Patent No. 4928693, of Goodin et al., issued May 29, 1990; however the device is not capable of perforating tissue using RF energy. U.S. Patent No. 6296615 B1, of Brockway et al., issued October 2, 2001, describes a catheter with a physiological sensor. This catheter consists of a pressure transducer for monitoring pressure, as well as the ability to detect and/or transmit an electrical signal.

Monitoring the ECG tracings from an intracardiac surgical device can also be a useful tool to verify the position of the device in a heart. An electrode with reference to a ground or a pair of electrodes placed directly on or in the heart will record a repeating pattern of changes in electrical 'action potential'. As action potentials spread from the top chambers of the heart (atria) to the bottom chambers of the heart (ventricles), the voltage measure between a single electrode and a ground or a pair of electrodes will vary in a way that provides a "picture" or electrocardiogram of the electrical activity of the heart. The nature of this picture can be varied by changing the position of the recording electrode(s); different locations in the heart are known to have characteristic ECG tracings or measurements. A bipolar recording is the voltage measurement between two electrodes and a unipolar recording is the voltage measurement between a single electrode and a reference electrode such as an electrode that is built into a recorder or electrocardiograph and maintained at zero potential (ground) or a reference electrode attached to a patient.

J. A. Alvarez et al. (1991) states that the endoatrial electrocardiogram registered while the needle (Brockenbrough transseptal needle) pressed muscular areas of the septum or the free atrial wall showed marked injury curves; on the other hand, no significant changes were observed at the area assumed to be the fossa ovalis floor. This implies that the ECG tracing observed when a surgical device is in contact with the fossa ovalis which is a membranous region in the atrial septum of a heart will be markedly different that the ECG tracing observed on the muscular areas of the atrial septum or the free atrial wall. This difference in the electrocardiogram may be used to locate a surgical device on the region of the fossa ovalis in a heart.

Knowing the electrical action potential or ECG at the tip of a surgical device is a useful tool to determine the location of the device, particularly in instances where imaging techniques provide inconclusive information. A device that is used for monitoring and recording the electrical activity of the heart is described in U.S. Patent No. 4892104, of Ito et al., issued January 9, 1990; however the device is not capable of perforating tissue using RF energy.

It is often required to create a perforation in the atrial septum to gain access to the left side of the heart interventionally to study or treat electrical or morphological abnormalities. It is also often desirable to create a hole in the septum in order to shunt the blood flow between the left and right sides of the heart to relieve high pressure or provide more blood flow to certain areas. The location on the atrial septum where a perforation is most commonly created is the fossa ovalis. The fossa ovalis is an oval depression in the inferior part of the interatrial septum. It represents the foramen ovale of the fetus and is generally a thin membraneous structure. Since the atrial septum is muscular in nature, it is generally easier to create a perforation across the fossa ovalis to gain access to the left side of a heart.

Historically in these instances, a dilator and guiding sheath are introduced into the femoral vein over a guidewire and advanced into the right atrium. The guidewire, dilator and guiding sheath are usually packaged as a kit with the guiding sheath designed to track over the dilator. In most designs, the distal end of the dilator extends out typically 4cm (about 1.57") beyond the distal end of the sheath once the two devices are locked together. Once the dilator and guiding sheath are positioned appropriately in the right atrium, a needle such as the Transseptal needle of Cook Incorporated, Bloomington, IN, USA is used. The needle comprises a stiff metal cannula with a sharpened distal tip. The needle is introduced through a dilator and guiding sheath set in the femoral vein and advanced through the vasculature into the right atrium. From there the needle tip is positioned at the fossa ovalis, the preferred location on the septum for creating a hole.

Once in position, the operator applies force at the proximal end of the needle and uses mechanical energy to advance the needle through the septum and into the left atrium. Once in the left atrium the needle can be attached to a pressure transducer and the operator can confirm a left atrial pressure before continuing with the procedure. An operator may dilate the hole by advancing the dilator over the needle into the left atrium and tracking the guiding sheath over the dilator and into the left atrium to provide access for other devices to the left heart once the needle and dilator are removed. As well, the operator may use another device such as a balloon catheter delivered over a guidewire to enlarge the hole made by the needle if a shunt between the right and left atria is desired.

Another device and method for creating a transseptal puncture is described in U.S. Patent No. 5403338, of Milo, issued April 4, 1995, which describes a punch that is intended to create an opening between two compartments. This device also makes use of mechanical energy, as with the transseptal needle.

These devices for and methods of creating a transseptal perforation rely on the skill of the operator and require practice to be performed successfully (Sethi et all, 2001). The needles used in this procedure are very stiff and can damage the vessel walls as they are being advanced. In addition, the amount of force required to perforate the septum varies with each patient. The force applied by the needle usually causes the septum to tent, or buckle, before it perforates the tissue. Once the needle makes the perforation, the needle may have significant forward momentum, which can be difficult to control. If too much force is applied there is the possibility of perforating the septum and continuing to advance the needle so far that damage is done to other areas of the heart. C.R. Conti (1993) discusses this possibility, and states that if the operator is not careful, the posterior wall of the heart can be punctured by the needle when it crosses the atrial septum because of the proximity of the two structures. It can also be difficult to position the needle appropriately in hearts that have malformations, or an atypical orientation. Justino et al. (2001) note that despite improvements to the technique with the needle since its first introduction, most large studies continue to report failed or complicated mechanical transseptal punctures, for reasons such as unusual septal thickness, or contour.

US Patent 6,565,562 "Method for the radio frequency perforation and the enlargement of a body tissue" issued to Shah et al. describes a method of perforating tissue such as an atrial septum using a radiofrequency (RF) perforating device. A functional tip on the RF perforating device is placed against target tissue and as RF current is applied a perforation is created. This method allows a perforation to be created without applying significant force that causes the tissue to tent and the RF perforating device easily passes through the tissue. However, even with this method there is danger of causing unwanted injury to other areas of the heart because the perforating device can be advanced too far unknowingly while RF current is being applied.

Patients requiring transseptal punctures would benefit from a device that decreases the risk of unwanted injury, which may include inadvertent puncture, perforation, laceration, or damage to cardiac structures. In particular, patients with a muscular septum, as well as those with a thick septum can benefit from an alternative to the transseptal needle puncture (Benson et al, 2002), as it is difficult to control the amount of mechanical force required to create the puncture. Furthermore, children born with heart defects such as hypoplastic left heart syndrome could benefit from an alternative technique. The abnormal anatomy of these patients including a small left atrium increases the likelihood of injury or laceration of surrounding structures during transseptal puncture (Sarvaas, 2002). The patient population discussed above would benefit from a device and technique for transseptal puncture that allows for a more controlled method of perforation and a method to confirm that the perforation has been made in the correct location.

### SUMMARY OF THE INVENTION

The present invention provides a surgical perforation method and device with one or more of ECG monitoring, pressure monitoring, a curve and staining abilities. The method and device relate to creating a perforation in material of a patient, including in particular, material that comprises the interatrial septum. The invention seeks to provide a device that is associated with a decreased risk of unintentional injury to other areas of the heart.

A device with a functional tip containing at least one active electrode capable of creating a controlled perforation in body tissue through the application of energy (e.g. Radio Frequency (RF)) is described. The position of the tip in a body can be determined in response to ECG measured at the tip and determined by a monitor coupled to the device. Alternatively or as well, the position of the tip can also be determined in response to pressure sensed at the tip and determined by a coupled monitor. The device is useful to remove or perforate unwanted tissue in a controlled manner in any location in the body, particularly in the atrial septum for controlled transseptal puncture. A portion of the distal region of the device is preferably curved in order to decrease the likelihood of injury to structures of a patient such as inadvertent perforation when used in transseptal perforation procedures. In this application, the device is introduced into the right atrium, and the functional tip is then positioned against the atrial septum. ECG is used to locate the region of the fossa ovalis on the atrial septum. Energy is applied to create the perforation. The tip is advanced toward the left atrium to create the perforation As it advances into the left atrium, for those embodiments having a curved shape, the device takes on its curved shape to direct the tip away from cardiac structures. Pressure, as well as ECG, may be monitored to determine if the perforation was created in a desired location.

Thus, the invention relates to a surgical device configured for decreasing the likelihood of unintentional cardiac injury. The curved portion of the distal region of the device is preferably constructed of an elastic material such that the distal region of the device may be made to conform to an internal lumen of a dilator in order to be advanced through the dilator within the vasculature. The distal end of the device remains inside the dilator while the energy delivery device is exposed and then positioned against the desired perforation location on the septum. This ensures that the device remains straight while it is being positioned and during perforation. Once the device has perforated the septum, it is advanced out of the guiding catheter and through the perforation. This allows the distal end to take on its natural curve within the left atrium. As a result of the curve the energy delivery device does not continue to move forward towards the posterior wall of the heart. The energy delivery device is positioned at the end of the curve, and is unlikely to inadvertently injure other structures within the heart. If the operator inadvertently advances the device beyond the desired location, the curved portion of the device, instead of the energy delivery device, would contact the posterior wall of the heart. The curved portion is constructed of a material that is unlikely to cause any damage to heart structures.

In accordance with a first aspect of the invention, there is provided a surgical device for cutting material and monitoring ECG. The device comprises: an elongate member having a distal region and a proximal region; an energy delivery device associated with the elongate member at the distal region for delivering cutting energy to the material, said energy delivery device adapted for connection to an energy source; and an ECG monitoring device associated with the distal region for monitoring ECG, said ECG monitoring mechanism adapted for connection to an ECG recording device.

In accordance with the first aspect a pressure sensing mechanism may be associated with the distal region for monitoring pressure about the distal region. The pressure sensing mechanism may comprise a pressure transmitting lumen extending between the proximal and distal regions. The lumen at the proximal region is adapted for fluid communication with a pressure transducer that provides a signal which varies as a function of pressure and adapted at the distal region for fluid communication with an environment about said distal region. The lumen is also adapted for injecting a fluid through at least one opening formed by the distal region.

In accordance with the first aspect, the distal region may comprise a portion having a curved shape to direct the energy delivery device in a desired direction. The portion may have a curved shape defining a radial arc. Further, the distal region may comprise a straight portion about 1 cm in length located distally of the portion having a curved shaped. Preferably, the proximal region comprises a marking indicative of the orientation of the portion having a curved shape.

In accordance with a second aspect, there is provided a surgical device for cutting material and monitoring pressure. The surgical device comprises: an elongate member having a distal region and a proximal region; an energy delivery device associated with the elongate member at the distal region for delivering cutting energy to the material, said energy delivery device adapted for connection to an energy source; and a pressure sensing mechanism associated with the distal region for monitoring pressure about the distal region.

In accordance with the second aspect the device may include an ECG monitoring device associated with the distal region for monitoring ECG about the distal region.

Further in accordance with the second aspect, the distal region may comprise a portion having a curved shape to direct the energy delivery device in a desired direction. Preferably, when the energy delivery device advances through the material, the distal region adopts a curved shape to direct the energy delivery device in a desired direction.

In accordance with a third aspect, a device for creating a perforation in material within a patient is provided. The device comprises: an elongate member having a proximal region and a distal region capable of adopting a curved shape; and a functional tip at the distal region for delivering energy to create the perforation in the material; wherein when the functional tip advances through the material, the distal region adopts a curved shape to direct the functional tip in a desired direction.

In accordance with the third aspect, the device may comprise one or both of a pressure sensing mechanism and an ECG monitoring device as previously described with reference to the first and second aspects.

In accordance with a fourth aspect, there is provided a device for creating a perforation in a heart septum comprising: an elongate member having a distal region and a proximal region; a functional tip at the distal region for delivering energy to create the perforation in the septum; and a control associated with the distal region and operable from the proximal region, wherein the control is operable to modify a shape of the distal region to direct the functional tip in a desired direction.

Also provided herein are method aspects. In accordance with a first method aspect, there is provided a method of surgery comprising the steps of: (i) introducing a surgical device into a heart of a patient, the surgical device comprising an elongate member having a distal region and a proximal region, an energy delivery device proximate to the distal region capable of cutting material and an ECG monitoring mechanism for determining ECG in the heart proximate to the distal region; (ii) positioning the energy delivery device to a first desired location in the heart adjacent material to be cut; (iii) delivering energy using the energy delivery device to cut said material; and (iv) monitoring ECG in the heart using the ECG monitoring mechanism in order to determine the position of the surgical device at least one of before and after step (iii).

In accordance with the first method aspect the method further comprises a step of: (v) moving the device to a second location. In one embodiment, the method may then further comprise a step of.: (vi) monitoring ECG using the ECG monitoring mechanism at the second location. In a further embodiment, the method may comprise (vi) monitoring pressure using a pressure sensing mechanism associated with the device at the second location.

In accordance with the first method aspect, preferably, step (ii) comprises dragging the surgical device about a surface of the heart while simultaneously monitoring ECG to determine the first desired location. The first desired location ma be determined in response to the' observation of a distinctive change in the ECG signal. In such a case, the ECG signal at the first desired location is damped in comparison with the ECG signal monitored otherwise on the surface of the heart as the surgical device is located about the first desired location. Further, the first desired location is a fossa ovalis of the heart.

In accordance with a second method aspect, there is a method of surgery comprising the steps of (i) introducing a surgical device into a body of a patient, the surgical device comprising an elongate member having a distal region and a proximal region, an energy delivery device proximate to the distal region capable of cutting material and a pressure sensing mechanism for determining pressure in the body proximate to the distal region; (ii) positioning the energy delivery device to a first desired location in the patient's body adjacent material to be cut; (iii) delivering energy using the energy delivery device to cut said material; and (iv) measuring pressure in the body using the pressure sensing mechanism in order to determine the position of the surgical device at least one of before and after step (iii).

In accordance with the first or second method aspects, step (i) comprises introducing the device into the patient's vasculature. The step of introducing the device into the patient's vasculature preferably comprises inserting the device into a dilator and a guiding sheath positioned in the patient's vasculature. In one embodiment, for either of the first and second method aspects, the method may then comprise a step of: (v) advancing the dilator and the sheath to a second location together over the surgical device. In another, the method may then'comprise a step of: (v) advancing the dilator, sheath and surgical device all together to a second location. Further, preferably the device and at least one of the dilator and the sheath comprise a radiopaque marking and step (ii) comprises aligning the radiopaque markings to aid in positioning the device.

In accordance with the first or second method aspects, step (ii) may comprise staining a region of tissue in the first desired location in the patient's body.

In accordance with the second aspect, step (ii) may comprise monitoring ECG using an ECG monitoring device associated with the distal region.

The second method aspect may further comprise a step of: (v) moving the device to a second desired location. In one embodiment, the method may then further comprise a step of: (vi) monitoring ECG using an ECG monitoring mechanism at the second location. In a further embodiment, the method may comprise (vi) monitoring pressure using the pressure sensing mechanism associated with the device at the second location.

In accordance with the first or second method aspects, the surgical device may comprise at least one depth marking and at least one radiopaque marker and step (v) of moving the surgical device to a second location comprise monitoring at least one of said depth markings and at least one of said radiopaque markers.

In accordance with the first or second method aspects, the distal region is capable of adopting a curved shape and the method comprises advancing a distal tip of the surgical device to a second location, directing the distal tip in a desired direction.

In accordance with a third method aspect there is provided a method of creating a perforation in a heart septum comprising: applying a form of energy to a surgical perforation device positioned at a desired location of a heart septum to create a perforation at the desired location, wherein the perforation device comprises an elongate member having a proximal region and a distal region capable of adopting a curved shape; and while advancing a distal tip of the device through the septum, directing the distal tip in a desired direction.

In accordance with the first, second or third method aspects, the surgical device comprises a control associated with the distal region and operable from the proximal region, wherein the control is operable to modify a shape of the distal region to direct the distal tip in a desired direction in relation to cardiac structures; and the method comprises operating the control.

In accordance with the third method aspect, the method may comprise manipulating the distal region to adopt the curved shape. In accordance with the third method aspect, the surgical perforation device comprises a pressure sensing mechanism for sensing pressure at the distal tip and the method comprises monitoring the pressure to indicate a location of the distal tip.

Preferably, according to any of the first, second or third method aspects, the surgical device comprises an orientation indicator for determining a direction of the distal tip and the method includes monitoring the orientation indicator to advance the distal tip in a desired direction.

It is to be understood that references to cut or cutting material such as tissue in relation to the present invention include perforating, ablating, coagulating and any other mechanism to create a void.

### DESCRIPTION OF THE DRAWINGS

In order that the invention may be readily understood, embodiments of the invention are illustrated by way of examples in the accompanying drawings, in which:
Figure 1 illustrates a schematic view of an electrosurgical system including an electrosurgical device in accordance with an embodiment of the invention;
Figure 2 illustrates a side cross-sectional view of the device of Figure 1;
Figure 3 illustrates a cross-sectional view of an alternate embodiment of the device;
Figure 4 illustrates an active electrode of the device of Figure 1;
Figure 5 illustrates the distal region of a device in accordance with an alternate embodiment of the invention;
Figure 6 illustrates a side cross-sectional view of an alternate embodiment of the device;
Figure 7 illustrates a side cross-sectional view of an alternate embodiment of the device;
Figures 8A and 8B illustrates a guiding sheath and dilator for use with the surgical device;
Figure 9 illustrates a first position of the device of Figure 1 against an atrial septum of a heart;
Figure 10 illustrates a second position of the device of Figure 1, after successful perforation of the atrial septum; and
Figures 11A and 11B illustrates a flow chart of a transseptal perforation method in accordance with this invention.

It will be noted that throughout the appended drawings, like features are identified by like reference numerals.

### DETAILED DESCRIPTION OF THE INVENTION

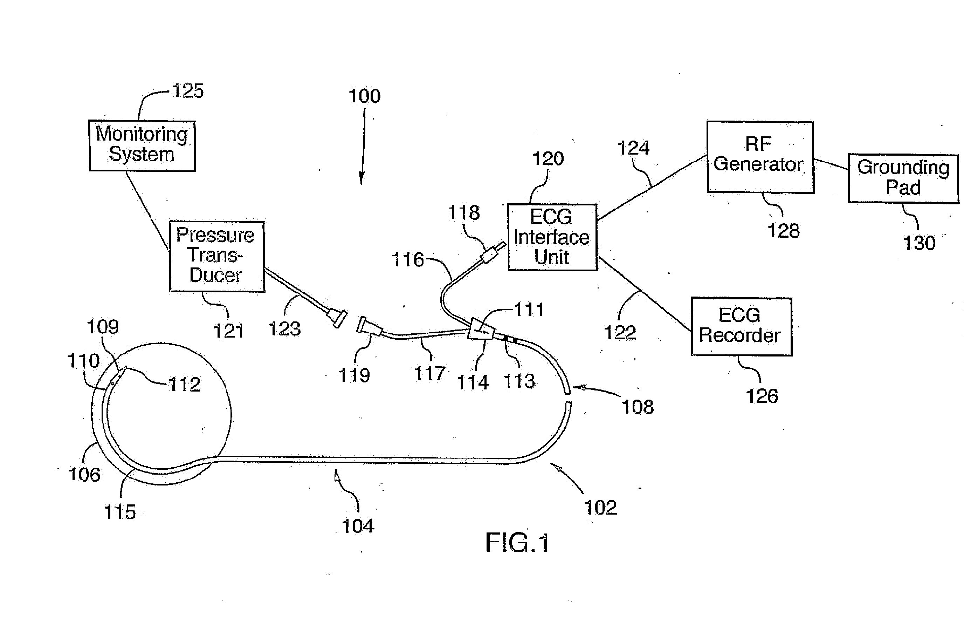

Figure 1 illustrates an embodiment of an electrosurgical perforation device 102 in accordance with the invention in an electrosurgical system 100. Device 102 comprises an elongate member 104 having a distal region 106, and a proximal region 108. Distal region 106 is adapted to be inserted within and along a lumen of a body of a patient, such as a patient's vasculature, and maneuverable therethrough to a desired location proximate to material such as tissue to be cut.

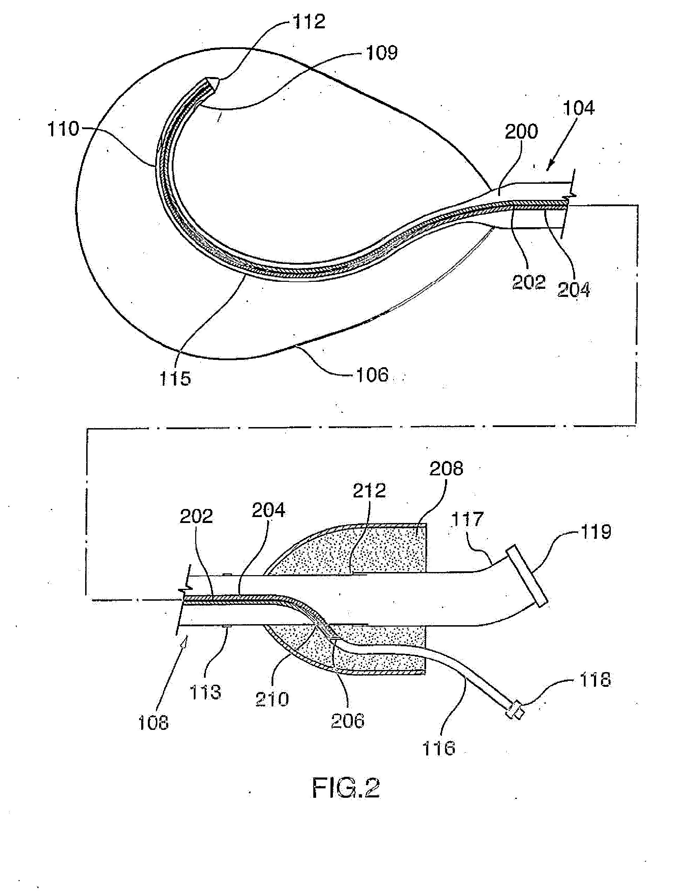

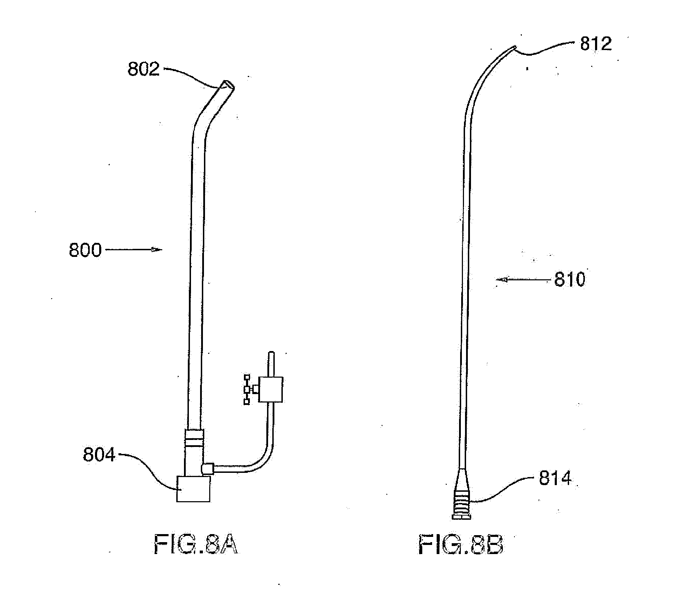

The elongate member 104 is typically tubular in configuration, having at least one lumen extending from proximal region 108 to distal region 106 such as lumen 200 shown in Figure 2. Elongate member 104 is preferably constructed of a biocompatible polymer material that provides column strength to device 102. The elongate member 104 is sufficiently stiff to permit a dilator 810 and a soft guiding sheath 800 (See Figure 8) to be easily advanced over device 102 and through a perforation. Examples of suitable materials for the tubular portion of elongate member 104 are polyetheretherketone (PEEK), and polyimide. In a preferred embodiment, the outer diameter of the tubular portion of elongate member 104 tapers down to connect to distal region 106. In alternate embodiments the outer diameter of elongate member 104 and the outer diameter of distal region 106 are the same.

Distal region 106 is constructed of a softer polymer material so that it is pliable and atraumatic when advanced through vasculature. The material is also formable, so that its shape can be changed during manufacturing, typically by exposing it to heat while it is fixed in a desired shape. In an alternate embodiment, the shape of distal region is modifiable by the operator during use. An example of a suitable plastic is Pebax (a registered trademark of Atofina Chemicals, Inc.). In the present embodiment, the distal region 106 comprises a curve portion 115 such that the distal region 106 curls up inside a patient's left atrium as the active electrode 112 crosses the patient's atrial septum. As the distal region 106 is advanced out of a guiding sheath, it curls away from the general axis of the sheath and the curve portion 115 assists to ensures that active electrode 112 is not in a position to inadvertently injure unwanted areas within a patient's heart after septal perforation. Preferably, curve length is approximately 5cm (about 2.26") and traverses 270 degrees of the circumference of a circle.

Preferably, curve portion 115 begins 1 cm (about 0.39") proximal to active electrode 112, leaving a 1 cm (about 0.39") distal portion of device 102 straight. This ensures that an initial portion of device 102 will exit dilator 810 (see Figure 8 below) straight, enabling the operator to easily position the device 102, for example, against a septum as described further below.

Distal region 106 preferably has a smaller outer diameter than elongate member 104 so that dilation of a perforation is limited while the distal region 106 is advanced through the perforation. Limiting dilation ensures that the perforation will not cause hemodynamic instability once device 102 is removed. The outer diameter of distal region 106 will preferably be no larger than 0.035" (0.897 mm). This is comparable to the distal outer diameter of the transseptal needle that is traditionally used for creating a perforation in the atrial septum. Elongate member 104 is preferably no larger than 0.050" (1.282 mm) which is also comparable to the transseptal needle dimensions.

Distal region 106 terminates at functional tip region 110 which comprises a device that functions as an energy delivery device as well as an ECG monitoring device. Functional tip region 110 comprises at least one active electrode 112 made of a conductive and radiopaque material, such as stainless steel, tungsten, platinum, or another metal. One or more radiopaque markings (not shown) may be affixed to elongate member 104 to highlight the location of the transition from distal region 106 to elongate member 104, or other important landmarks on device 102. Alternately, the entire distal region 106 of device 102 may be radiopaque. This can be achieved by filling the polymer material, Pebax used to construct distal region 106 with a radiopaque filler. An example of a suitable radiopaque filler is Bismuth. Distal region 106 defines at least one opening 109 in fluid communication with main lumen 200 (Figure 2) as described further below.

Proximal region 108 comprises a hub 114, to which are attached a catheter connector cable 116, and connector 118. Tubing 117 and adapter 119 are attached to hub 114 as well. Proximal region 108 may also have one or more depth markings 113 to indicate distances from functional tip region 110, or other important landmarks on device 102. Hub 114 comprises a curve direction or orientation indicator 111 that is located on the same side of device 102 as the curve 115 in order to indicate the direction of curve 115. Orientation indicator 111 may comprise inks, etching, or other materials that enhance visualization or tactile sensation. Persons of ordinary skill in the art will appreciate that one or more curve direction indicators may be used and that they may be of any suitable shape and size and a location thereof may be varied about the proximal region 108.

Adapter 119 is configured to releaseably couple device 102 to an external pressure transducer 121 via external tubing 123. External pressure transducer 121 is coupled to a monitoring system 125 that converts a pressure signal from external pressure transducer 121 and displays pressure as a function of time. Catheter connector cable 116 connects to ECG interface unit 120 via connector 118. ECG connector cable 122 connects ECG interface unit 120 to ECG recorder 126 which displays and captures ECG signals as a function of time. Generator connector cable 126 connects ECG interface unit 120 to an energy source such as Generator 128. ECG interface unit 120 functions as a splitter, permitting connection of electrosurgical device 102 to both ECG recorder 126 and RF generator 128 at the same time. ECG signals can be continuously monitored and recorded and the filtering circuit (not shown) within ECG interface unit 120 permits RF energy to be delivered from RF generator 128 through electrosurgical device 102 without compromising the ECG recorder 126.

Generator 128 is preferably a radiofrequency (RF) electrical generator that is designed to work in a high impedance range. Because of the small size of active electrode 112 the impedance encountered during RF energy application is very high. General electrosurgical generators are typically not designed to deliver energy in these impedance ranges, so only certain RF generators can be used with this device. In the preferred embodiment, the energy is delivered as a continuous wave at a frequency between about 400 kHz and about 550 kHz. An appropriate generator for this application is the BMC RF Perforation Generator (model number RFP-100, Baylis Medical Company, Montreal, Canada). This generator delivers continuous RF energy at about 460 kHz. A grounding pad 130 is coupled to generator 128 for attaching to a patient to provide a return path for the RF energy when generator 128 is operating in a monopolar mode. Other embodiments could use pulsed or noncontinuous RF energy. In still other embodiments of the electrosurgical perforation device 102, different energy sources may be used, such as microwave, ultrasound, and laser with appropriate energy delivery coupling devices and energy delivery devices.

Referring to Figure 2 a cross-section of device 102 is illustrated in accordance with the embodiment of Figure 1. Functional tip region 110 comprises an active electrode 112 that is coupled to an insulated conducting wire 202. Conducting wire 202 is preferably attached to distal region 106 using an adhesive. Alternately, distal region 106 is melted onto insulation 204 on conducting wire 202 to form a bond.

Conducting wire 202 carries electrical energy from generator 128 to the active electrode 112. Conducting wire 202 also carries action potentials or voltage measured by active electrode 112 to ECG recorder 126. Action potentials or voltage measured by active electrode 112 is with reference to a zero potential or ground electrode (not shown) within ECG recorder 126 or with reference to a ground electrode (not shown) attached to the patient (not shown). Conducting wire 202 is covered with electrical insulation 204 made of a biocompatible material that is able to withstand high temperatures such as polytetrafluoroethylene (PTFE), or other insulating material. Conducting wire 202 preferably extends through a main lumen 200 of device 102 which lumen extends from proximal region 108 to distal region 106.

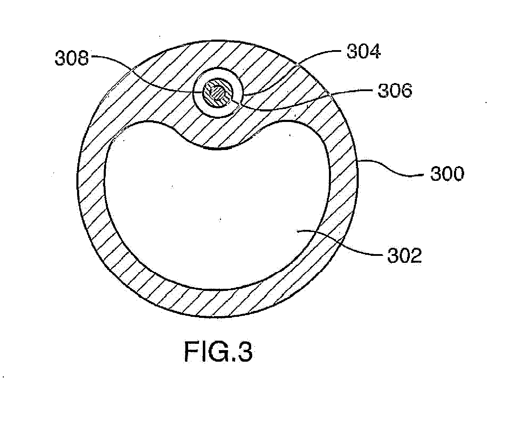

In an alternate embodiment shown in cross section view in Figure 3, an elongate member 300 comprises main lumen 302 and a separate lumen 304. The separate lumen 304 contains a conducting wire 306 covered with electrical insulation 308 therein and main lumen 302 can be used for aspiration of blood and injection of contrast (e.g. for staining) and other media. This embodiment of elongate member 300 allows a dedicated lumen for each function of device 102.

In the embodiment of Figure 2, main lumen 200 extends from proximal region 108 along elongate member 104 and through distal region 106 of device 102. At least one opening 109 at the distal region 106 provides a pathway between main lumen 200 and the environment surrounding distal region 106, such as a desired location within a patient's body. Openings 109 are sufficiently dimensioned to easily aspirate blood to and through main lumen 200 and to inject radiopaque contrast; however, openings 109 are limited in number and dimension so that they do not compromise the structural integrity of distal region 106. In order to facilitate even distribution of contrast agent and to prevent pooling in main lumen 200 at distil region 106, openings 109 may be dimensioned such that distally located openings (not shown) are larger than proximally located openings (not shown). The location of openings 109 is as close to active electrode 112 as possible so that only a small portion of device 102 is required to be proximate to the desired location for the determination of pressure.

Adapter 119 is configured for releaseably coupling to an external pressure transducer 121, or a standard syringe. Preferably, adapter 119 comprises a female Luer lock connection. Adapter 119 is coupled to main lumen 200 via tubing 117 to provide a pathway from main lumen 200 to external pressure transducer 121 so that blood pressure can be determined using a method that is known to those of ordinary skill in the art. Conducting wire 202 exits elongate member 104 through an exit point 210. Exit point 210 is sealed with an adhesive or a polymeric material. Conducting wire 202 extends along elongate member 104 from distal region 106 to proximal region 108 and is electrically coupled to catheter connector cable 116 within hub 114 by an electrical joint 206. This joint can be made by soldering, or another wire joining method known to people of ordinary skill in the art. Catheter connector cable 116 terminates with a connector 118 that can mate with either the ECG interface unit 120, or a separate extension connector cable (not shown). Catheter Connector cable 116 and connector 118 are nade of materials suitable for sterilization, and will insulate the user from energy traveling through the conductor.

Elongate member 104 is coupled to tubing 117 at proximal end 212 of elongate member 104. Tubing 117 is made of a polymeric material that is more flexible than elongate member 104. A suitable material for tubing 117 is polyvinylchloride (PVC), or another flexible polymer. Tubing 117 is coupled to adapter 119. This configuration provides a flexible region for the user to handle when releaseably coupling external pressure transducer 121, or other devices to adapter 119. Couplings between elongate member 104 and tubing 117, and tubing 117 and adapter 119 are made with an adhesive such as a UV curable adhesive, an epoxy, or another type of adhesive.

A hub 114 surrounds electrical joint 206 and proximal end of elongate member 104 in order to conceal these connections. The hub 114 is made of a polymeric material, and is filled with a filling agent 208 such as an epoxy, or another polymeric material in order to hold catheter connector cable 116 and tubing 117 in place.

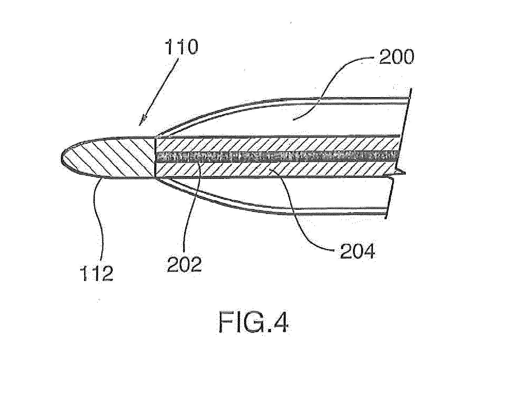

Referring to Figure 4 there is illustrated a side cross-sectional view of an embodiment of functional tip region 110. Functional tip region 110 comprises one active electrode 112 configured in a bullet shape. Active electrode 112 is preferably 0.059" (0.15cm) long and preferably has an outer diameter of 0.016" (0.04cm). Active electrode 112 is coupled to an end of conducting wire 202, also made out of a conductive and radiopaque material. RF energy is delivered through active electrode 112 to tissue, and travels through the patient to grounding pad 130, which is connected to generator 128. Additionally, action potentials or voltage measured from tissue through active electrode 112 travel through conducting wire 202 to ECG recorder 126. Alternate embodiments of active electrode 112 are configured in shapes other than a bullet. These shapes include a spherical shape, a rounded shape, a ring shape, a semi-annular shape, an ellipsoid shape, an arrowhead shape, a spring shape and a cylindrical shape, among others.

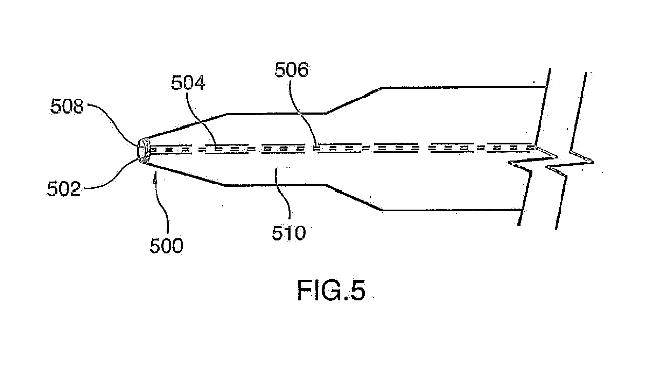

Referring to Figure 5 there is illustrated an alternate embodiment of a functional tip region 500. Functional tip region 500 comprises one active electrode 502 in a ring configuration. Conducting wire 504 covered with electrical insulation 506 is coupled to the active electrode 502, and active electrode 502 is positioned around a perimeter of a single opening 508 that provides a pathway between main lumen 510 and a patient's body. Another similar embodiment to functional tip region 500 comprises an active electrode in a partially annular shape (not shown). In other embodiments (not shown), a functional tip comprises multiple electrodes. Such electrodes may operate in a monopolar mode as with the embodiments detailed in Figures 2 and 5.

In order to measure pressure at the distal region 106 of the device 102, an external pressure transducer 121 may be coupled to device 102. For example, adapter 119 is releaseably coupled to external tubing 123 that is coupled to external pressure transducer 121 as shown in Figure 1. External tubing 123 is flushed with saline to remove air bubbles. When device 102 is positioned in a blood vessel in a body, pressure of fluid at distal region 106 exerts pressure through openings 109 on fluid within main lumen 200, which exerts pressure on saline in external tubing 123, which exerts pressure on external pressure transducer 121. The at least one opening 109 and lumen 200 provide a pressure sensing mechanism in the form of a pressure transmitting lumen for coupling to pressure transducer 121. External pressure transducer 121 produces a signal that varies as a function of the pressure it senses. External pressure transducer 121 is also releaseably electrically coupled to a pressure monitoring system 125 that converts the transducer's signal and displays a pressure contour as a function of time. Thus, pressure may be optionally measured and/or recorded and, in accordance with a preference of a method aspect as described further herein below, used to determine a position of the distal region 106 in a patient's body.

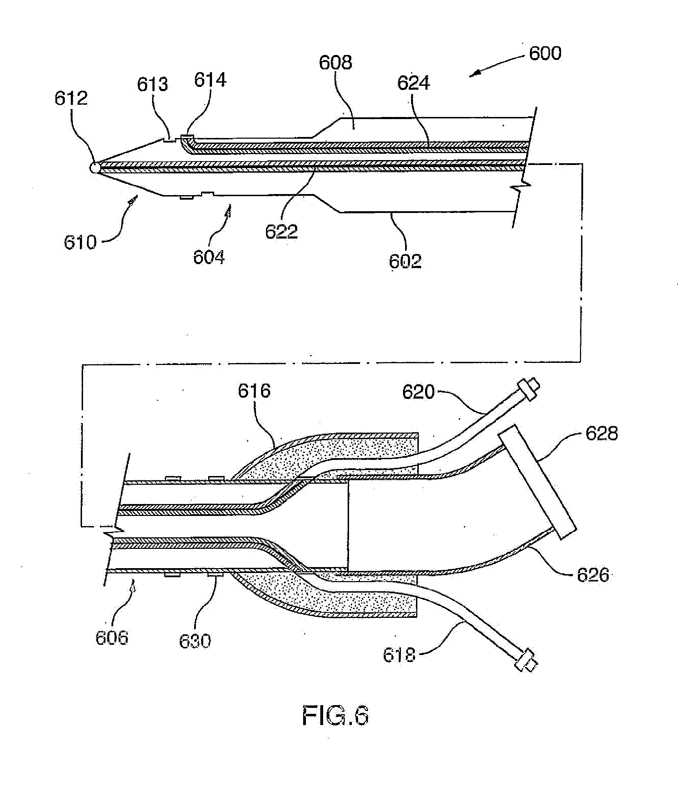

Referring to Figure 6, there is illustrated a side cross-sectional view of an alternate embodiment of device 600 which operates in a bipolar mode. Device 600 comprises an elongate member 602 having a distal region 604, and a proximal region 606. Elongate member 602 has at least one lumen 608 extending from proximal region 606 to distal region 604. The outer diameter of elongate member 602 tapers down to connect to distal region 604. In alternate embodiments the outer diameter of elongate member 602 and the outer diameter of distal region 604 are the same.

Distal region 604 terminates at functional tip region 610. Functional tip region 610 comprises one active electrode 612 and one reference electrode 614. In an alternate embodiment comprising a set including device 600 and at least one of a sheath, such as sheath 800 and a dilator, such as dilator 810 of Figure 8, a reference electrode (not shown) may be located at the distal tip 812 of dilator 810 or at the distal tip' 802 of sheath 800. Both the active electrode 612 and reference electrode 614 can be configured in many different shapes. These shapes include a spherical shape, a rounded shape, a ring shape, a semi-annular shape, an ellipsoid shape, an arrowhead shape, a spring shape and a cylindrical shape, among others. One or more radiopaque markings (not shown) may be affixed to elongate member 602 to highlight the location of the transition from distal region 604 to elongate member 602, or other important landmarks on device 600. Alternately, the entire distal region 604 of device 600 may be radiopaque. Distal region 604 defines at least one opening 613 in fluid communication with lumen 608.

Lumen 608 extends from proximal region 606 along elongate member 602 and through distal region 604 of device 600. At least one opening 613 at the distal region 604 provides a pathway between lumen 608 and the environment surrounding distal region 604, such as a desired location within a patient's body. Openings 613 are sufficiently dimensioned to easily aspirate blood to and through lumen 608 and to inject radiopaque contrast; however, openings 613 are limited in number and dimension so that they do not compromise the structural integrity of distal region 604. In order to facilitate even distribution of contrast agent and to prevent pooling in lumen 608 at distal region 604, openings 613 may be dimensioned such that distally located openings (not shown) are larger than proximally located openings (not shown). The location of openings 613 is as close to active electrode 612 as possible so that only a small portion of device 600 is required to be proximate to the desired location for the determination of pressure.

Proximal region 606 comprises a hub 616, an active connector cable 618, a reference connector cable 620, tubing 626 and an adapter 628. Proximal region 606 may also have one or more depth markings 630 to indicate distances from active electrode 612, or other important landmarks on device 600. Adapter 628 is configured to releaseably couple device 600 to an external pressure transducer (not shown). Both active connector cable 618 and reference connector cable 620 connect to ECG interface unit (not shown).

Active electrode 612 is coupled to an insulated conducting wire 622. Conducting wire 622 carries electrical energy from a generator (not shown) to the active electrode 612. Conducting wire 622 also carries action potentials or voltage measured by active electrode 612 to an ECG recorder (not shown). Conducting wire 622 extends through main lumen 608 of device 600. Conducting wire 622 extends along elongate member 602 from distal region 604 to proximal region 606 and is electrically coupled to active connector cable 618 within hub 616.

Reference electrode 614 is coupled to an insulated conducting wire 624. Conducting wire 624 carries electrical energy from a patient (not shown) to a generator (not shown). Conducting wire 624 also carries action potentials or voltage measured by reference electrode 614 to an ECG recorder (not shown). Conducting wire 624 extends through main lumen 608 of device 600. Conducting wire 624 extends along elongate member 602 from distal region 604 to proximal region 606 and is electrically coupled to reference connector cable 620 within hub 616.

In the bipolar mode, RF energy is delivered through active electrode 612, and returns to the generator through reference electrode 614. The use of an active and a reference electrode attached to device 600 eliminates the need for a grounding pad to be attached to the patient as is well understood by persons of ordinary skill in the art. With an active-return electrode arrangement at functional tip region 610, action potentials or voltage measured by the active electrode 612 are with reference to the ground or reference electrode 614 located at the function tip region 610. The ECG recorder assigns a zero potential value to the reference electrode 614. A zero potential or ground electrode within the ECG recorder or placement of a ground electrode on the patient is not required and a higher fidelity recording may be facilitated.

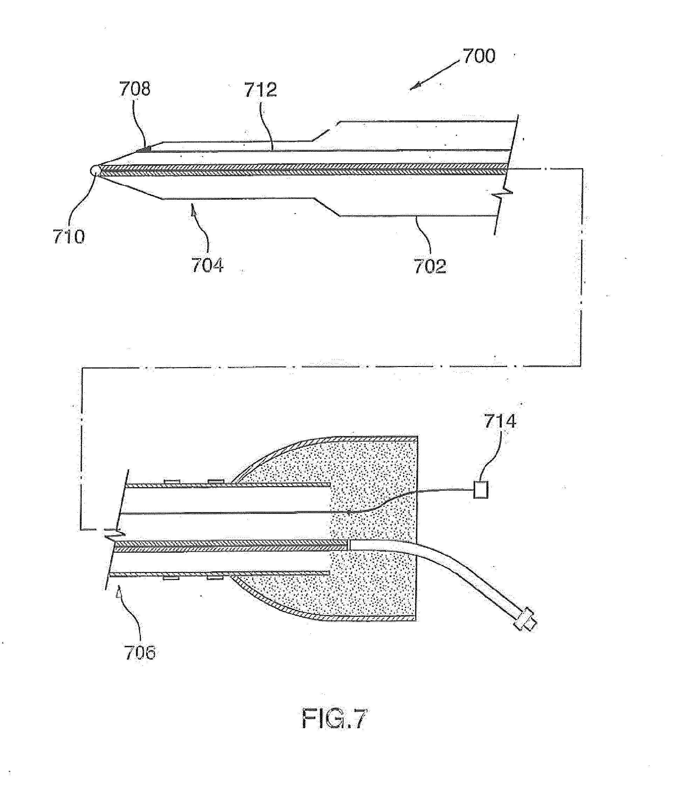

Referring to Figure 7 there is illustrated a side cross-sectional view of proximal 706 and distal 704 regions of an alternate embodiment of an electrosurgical perforation device 700 that does not require an external pressure transducer. In this embodiment the pressure sensing mechanism comprises an on-board pressure transducer 708 coupled by an adhesive to elongate member 702 at distal region 704. The pressure transducer 708 is configured at distal region 704 such that pressure close to active electrode 710 can be transduced. The on-board pressure transducer 708 is electrically coupled to a pressure communicating cable 712 to provide power to transducer 708 and to carry a pressure signal to proximal region 706 of the electrosurgical perforation device 700. Pressure communicating cable 712 terminates in a monitoring system connector 714 that is configured to be releaseably coupled to pressure monitoring system (not shown). The pressure monitoring system converts the pressure signal and displays pressure as a function of time. In the embodiment of Figure 7, a main lumen such as the main lumen 200 of Figure 2 is not required for fluid communication with an external pressure transducer 121 (shown in Figure 1). In addition, this embodiment does not require openings, such as openings 109 shown in Figure 1, at distal region 704 for fluid communication with a main lumen. However, a lumen with openings may be provided for injecting or aspirating fluids, if desired. Optionally, to measure and record ECG at the distal region of the device 102, ECG recorder 122 is connected to device 102 through the ECG interface unit 120. Hub 114 is coupled to catheter connector cable 116 that is coupled to connector 118 as shown in Figure 1. Connector 118 is attached to ECG Interface unit 120. When device 102 is maneuvered in a patient's body, particularly in a heart, electrical action potentials or voltage detected by active electrode 112 are transmitted along conducting wire 202 and catheter connector cable 116, through ECG interface unit 120 and are captured and displayed on ECG recorder 126. Different locations in a heart are at different potentials and the voltage measured varies as the position of active electrode 122 is varied. A conversion circuit (not shown) within ECG recorder 126 converts the measured voltage or potential into a picture or waveform recording that varies as a function of time.

Device 102 of this invention or alternate embodiments can be used for general electrosurgery in instances where it is desirable to cut tissue or other material and monitor ECG, pressure or both. More specifically, it can be used for creating a perforation such as a transseptal perforation. In order to create a perforation in the heart, device 102 is delivered to the heart using a guiding sheath and dilator known to those of ordinary skill in the art. Figures 8A and 8B show an embodiment of a guiding sheath 800 and a dilator 810 respectively. Guiding sheath 800 has a tip 802 at the distal end and a proximal hub 804. Guiding sheath 800 has a lumen (not shown) through which dilator 810 is most commonly delivered. Guiding sheath 800 may have a radiopaque marker (not shown) located distally. Guiding sheath 800 may have a reference electrode (not shown) located distally. Dilator 810 has a tip 812 at the distal end and a proximal hub 814. Dilator 810 may have a radiopaque marker (not shown) located distally. Dilator 810 may have a reference electrode (not shown) located distally. Dilator 810 has a lumen (not shown) through which a guidewire (not shown) or electrosurgical perforation device 102 can be delivered.

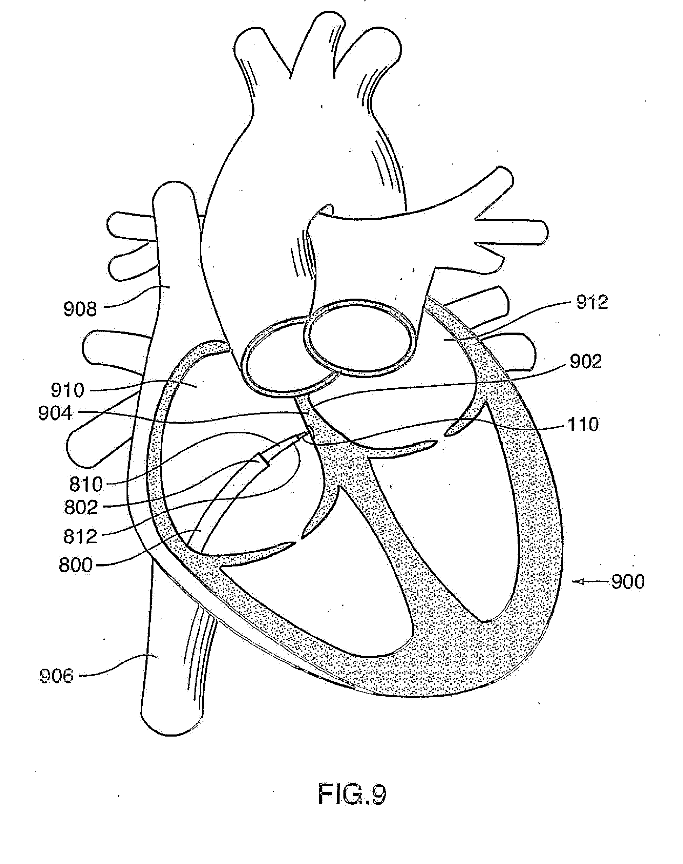

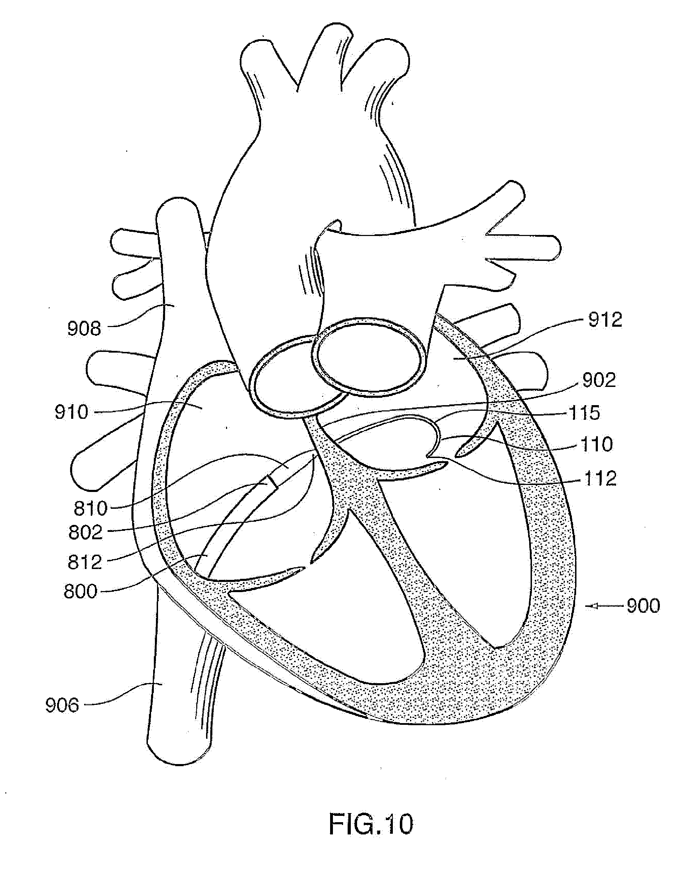

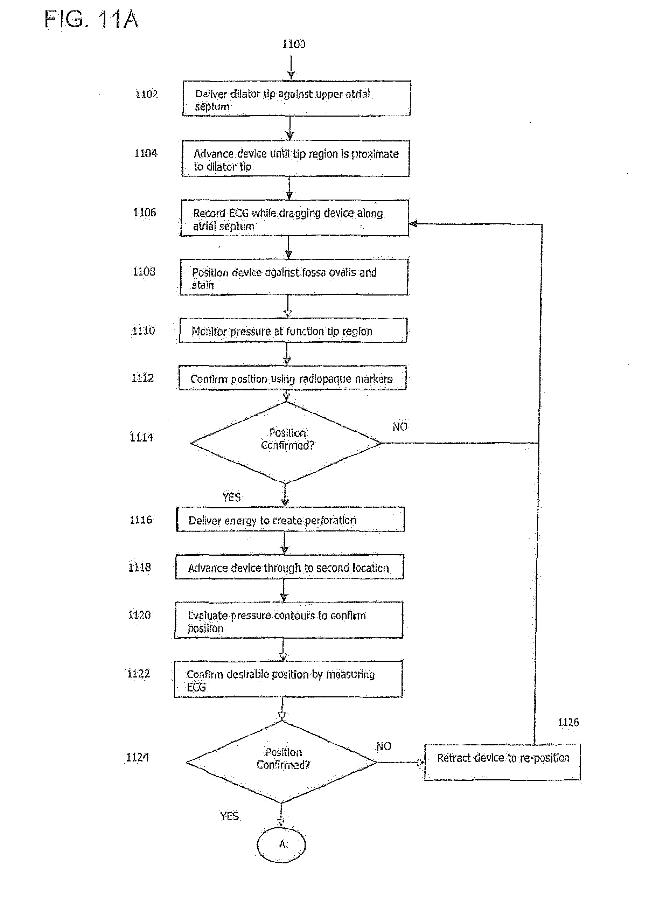

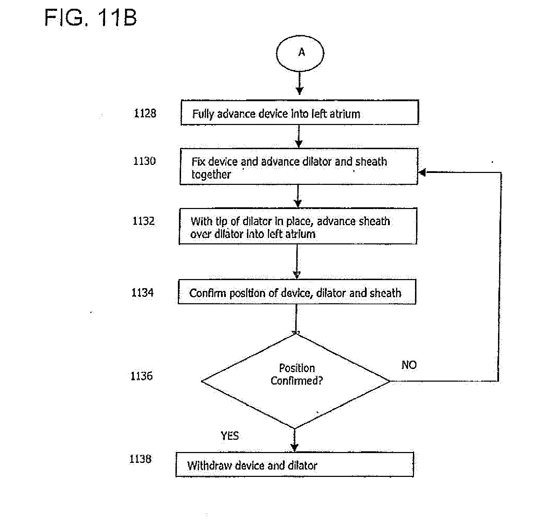

Referring to Figure 9 and 10 there is illustrated electrosurgical perforation device 102 inserted through a dilator 810 and sheath 800 within a heart 900 of a patient. Operational steps 1100 for a method of creating a transseptal perforation in accordance with an embodiment of the invention are outlined in flowchart form in Figures 11A and 11B. In accordance with a method aspect of the invention for creating a transseptal perforation, to deliver the tip 812 of the dilator 810 against the upper region of the atrial septum 902 (step 1102) a guiding sheath 800 and dilator 810 with a lumen sufficient to accommodate the outer diameter of the electrosurgical perforation device 102 is introduced into a patient's vasculature. Guiding sheath 800 and dilator 810, known to those of ordinary skill in the art, are advanced together through the vasculature, approaching the heart from the Inferior Vena Cava 906, into the Superior Vena Cava (SVC) 908 of the heart 900. The sheath 800 and dilator 810 are withdrawn from the SVC 908, into a right atrium 910, and contrast agent is delivered through dilator 810 while positioning the dilator 810 and sheath 800 along an atrial septum 902. The sheath 800 and dilator 810 are now positioned within the right atrium 910 of heart 900 so that the tip 812 of dilator 810 is located against the upper region of the atrial septum 902(step 1102).

Once the tip 812 of dilator 810 is in position against the upper region of the atrial septum 902, device 102 can be advanced through the dilator 810 until functional tip region 110 is located distally to tip 812 of dilator 810 (step 1104). Distal region 106 of device 102 is pliable so that the curve 115 straightens out within the dilator 810 and takes on the shape of the dilator 810 as it is advanced to the atrial septum 902. Device 102 is coupled to the ECG recorder 126 and an ECG tracing monitored through active electrode 112, known to those of ordinary skill in the art, may be shown on ECG recorder 126. The technique for obtaining an ECG tracing was previously described. Device 102, dilator 810, and guiding sheath 800 are now dragged along the atrial septum 902 while monitoring the ECG tracing on the ECG recorder 126 (step 1106). Confirmation of position of active electrode 112 of device 102 against the fossa ovalis 904 is made once a distinctive change in the ECG tracing on ECG recorder 126 is observed. This is due to active electrode 112 advancing over the region of the fossa ovalis 904 which is membranous in comparison with the muscular atrial septum 902.

J. A. Alvarez et al. (1991) who performed experiments on the usefulness of the intracavitary ECG (recorded using a transseptal needle) in the localization of the fossa ovalis states that when the tip of the needle was laid against the fossa ovalis floor, the endoatrial electrocardiogram registered a slight or no injury curve, even when the pressure was sufficient to perforate the septum. On the contrary, the pressure on any other areas of the muscular septum or atrial walls elicited a bizarre monophasic injury curve. This shows that the ECG signal recorded by a surgical device while on the membranous fossa ovalis will be damped in comparison with the ECG signal recorded on the muscular areas of the atrial septum or atrial walls. This difference in ECG signal may be useful in locating the region of the fossa ovalis as a surgical device is positioned within a heart. ECG may be observed on a screen (not shown) and printed on a chart (not shown), for example. The distinctive change may be signaled for observation as well using an alarm such as a sound or light signal.

Functional tip region 110 is now directed toward the fossa ovalis 904, a preferred first desired location on the atrial septum 902 to create a perforation. Contrast agent delivered through device 102 will be directed through the tip 812 of dilator 810 directly into the tissue of the fossa ovalis 904 staining it radiopaquely (Figure 9 and step 1108 of Figure 11A). Under fluoroscopy, the stained region of the fossa ovalis 904 can be seen as a dark patch on a lighter gray colored atrial septum 902.

The position of device 102 may also be confirmed by monitoring pressure at the functional tip region 110 (step 1110). Device 102 is coupled to external pressure transducer 121 and a right atrial pressure contour, known to those of ordinary skill in the art, may be shown on monitoring system 125. The technique for obtaining a pressure contour was previously described.

The position of functional tip region 110 and active electrode 112 may be additionally confirmed using an imaging modality such as fluoroscopy. Under fluoroscopy the radiopaque markings (not shown) associated with distal region 106 of device 102 may be aligned with the radiopaque marker (not shown) located distally on dilator 810 such that functional tip region 110 of device 102 is located at the fossa ovalis 904. Alternately, the radiopaque markings (not shown) associated with distal region 106 of device 102 may be aligned with the radiopaque marker (not shown) located distally on sheath 800 such that functional tip region 110 of device 102 is located at the fossa ovalis 904 (step 1112). The position is evaluated and if the desired position is not confirmed (step 1114, No branch), step 1106 may be repeated. If confirmed (step 1114, Yes branch), energy may be delivered to create the perforation. For example, generator 128 is activated and RF energy is delivered through device 102 to make a perforation (step 1116).

The functional tip region 110 of device 102 is thereafter advanced through the perforation and into a second location (step 1118). Advancement may be monitored under fluoroscopy using the radiopaque markings (not shown) on the distal region 106 of device 102. The preferred second location is left atrium 912 of the heart. The distal region 106 of device 102 is advanced incrementally into the left atrium 912 through dilator 810, for example, in 1cm (about 0.39") increments. After the first 1cm of distal region 106 of device 102 has been advanced out of dilator 810 across the atrial septum 902, into left atrium 912, distal region 106 of device 102 establishes its curved shape within the left atrium 912. The position of depth markings 113 of device 102 relative to proximal hub 814 of dilator 810 can be used as a guide. Additionally, advancement of perforating device 102 can be controlled by monitoring the radiopaque markings (not shown) on the distal region 106 of device 102 under fluoroscopy. When the openings 109 on distal region 106 of device 102 are located in the left atrium 912, the evaluation of pressure contours from the left atrium via pressure transducer 121 (step 1120) can be performed. Device 102 preferably remains coupled to external pressure transducer 121 so that a pressure contour at the second location can be monitored confirming the desired location of the dital region following the perforation.

Additionally, when the distal region 106 of device 102 is located in the left atrium 912, the evaluation of the ECG tracing (step 1122) can be performed. Device 102 remains coupled to ECG recorder 126 so that an ECG tracing at the second location can be monitored.

After successful perforation, a left atrial pressure contour known to those of ordinary skill in the art, will be shown on the monitoring system 125. As well a left atrial ECG tracing, known to those of ordinary skill in the art, will be shown on the ECG recorder 126. In the event that the imaging, pressure contours and ECG tracings show that the perforation is made in an undesirable location (step 1124, No branch), device 102 is retracted into the right atrium 910 (step 1126) and is repositioned for another perforation attempt (step 1106). If the perforation is successfully made in the correct location (step 1124, Yes branch), distal region 106 of device 102 is preferably further advanced through the perforation. When device 102 is fully inserted into the dilator 810, hub 114 of the device 102 will be flush against proximal hub 814 of dilator 810, and no depth markings 113 of device 102 will be visible (step 1128, Figure 11B). When fully inserted, device 102 provides sufficient support to permit the dilator 810 to be advanced over it through the perforation.

Hub 114 of device 102 may be fixed in place spatially, and both the proximal hub 814 of dilator 810 and proximal hub 804 of sheath 800 are incrementally advanced forward, together, thus sliding the dilator 810 and sheath 800 over device 102 (step 1130). The tip 812 of dilator 810 and the tip 802 of sheath 800 are monitored under fluoroscopy as they are advanced over device 102 and once the tip 812 of dilator 810 has breeched the perforation, and advanced into the left atrium 912, the tip 802 of sheath 800 is advanced over dilator 810, across the perforation and into the left atrium 912 (step 1132).

In an alternate method of advancing the sheath and dilator into the left atrium, (not shown), once distal region 106 is fully advanced through the perforation and into the left atrium 912, and hub 114 of device 102 is flush against proximal hub 814 of dilator 810, hub 114 of device 102 and proximal hub 814 of dilator 810 and proximal hub 802 of sheath 800 may all be advanced forward together, preferably under fluoroscopy. Forward momentum will cause the tip 812 of dilator 810 to breech the perforation, advancing into the left atrium 912. The tip 802 of sheath 800 will follow over dilator 810, across the perforation and into the left atrium 912.

At step 1134, the positions of distal region 106 of device 102, tip 812 of dilator 810 and tip 802 of sheath 800 are confirmed, for example, under fluoroscopy to be in the left atrium 912. If not in the desired location (step 1136), step 1130 may be repeated. If the positions are confirmed (step 1136), device 102 and dilator 810 may now be respectively withdrawn outside the body, preferably under fluoroscopic guidance (step 1138). While maintaining the position of tip 812 of dilator 810 and tip 802 of sheath 800 in the left atrium 912, device 102 may be withdrawn. Dilator 810 may now be withdrawn outside the body under fluoroscopic guidance, while maintaining the position of the tip 802 of sheath 800 in the left atrium 912. Optionally, a contrast agent may now be injected through sheath 800 into the left atrium 912, or blood aspirated through sheath 800 from the left atrium 912 and sheath 800 may now be used to deliver other catheters (not shown) to the left atrium 912.

The present invention in various aspects thus provides a device and method that is capable of creating a controlled perforation while determining a position of the device in response to action potentials or measured voltage at a location in the heart as well as determining a position of the device in response to pressure at a location in the body. The present invention minimizes the risk of inadvertent cardiac injury while creating the perforation. The present invention also provides a method for staining the area to be perforated in order to make it easier to locate during the perforation. In addition, the present invention provides a method for delivering a dilator and sheath over the device after the perforation. The controlled perforation is created by the application of energy by a generator to an active tip on the device. A preferred means for minimizing the risk of inadvertent injury comprises a curve at the distal end of the device. A means for determining the position of the device may comprise monitoring action potentials or measured voltage through an active electrode in a unipolar or bipolar manner and displaying the ECG tracings on an ECG recorder. In this embodiment there is at least one active electrode at the functional tip region for monitoring action potentials which are captured and displayed as ECG tracings on an ECG recorder. A means for determining the position of the device may also comprise a pressure transmitting lumen that can be releasably coupled to an external pressure transducer. In this embodiment, there is at least one opening near the distal region of the device for blood or other fluid to enter and fill the lumen and exert a measurable pressure on a coupled external transducer. The lumen and opening may also be useful for injecting radiopaque contrast or other agents through the device. In an alternate embodiment, the means for determining a position of the device in response to pressure comprises a transducer located on the device proximal to the functional tip.

The device of the invention is useful as a substitute for a traditional transseptal needle to create a transseptal perforation. The device of the present invention preferably has a soft and curved distal region with a functional tip that uses RF energy to create a perforation across a septum, making the procedure more easily controlled and less operator dependent than a transseptal needle procedure. The soft distal region of the device reduces incidents of vascular trauma as the device is advanced through the vasculature. The application of RF energy is controlled via an electric generator, eliminating the need for the operator to subjectively manage the amount of force necessary to cross the septum with a traditional needle. The present invention eliminates the danger of applying too much mechanical force and injuring the posterior wall of the heart.

The present invention also provides a method for the creation of a perforation in an atrial septum. ECG as well as pressure monitoring is particularly important in this procedure, as there is the possibility of inadvertently perforating the aorta due to its proximity to the atrial septum. Electrical action potential or voltage measurements displayed as ECG tracings allow the operator to position the device accurately at the fossa ovalis on the septum as well as confirm that the distal end of the device has entered the left atrium, and not the aorta, or another undesirable location in the heart. As well, pressure measurements allow the operator to confirm that the distal end of the device has entered the left atrium, and not the aorta, or another undesirable location in the heart. Staining the atrial septum is also particularly important in this procedure, as it easily identifies the region of the atrial septum (fossa ovalis) to be perforated. Preferably, the device will also be visible using standard imaging techniques; however the ability to monitor both ECG and pressure provides the operator with a level of safety and confidence that would not exist using only these techniques.

The present invention also provides a method for delivering the dilator and sheath over the electrosurgical perforation device into the left atrium once a successful perforation has been created. One of the main reasons for creating a transseptal perforation is to gain access to the left side of the heart for delivery of catheters or devices to treat left-sided heart arrhythmias or defects.

While the surgical device thus described is capable of cutting living tissue, it will be understood by persons of ordinary skill in the art that an appropriate device in accordance with the invention will be capable of cutting or removing material such as plaque or thrombotic occlusions from diseased vessels as well.

Persons of ordinary skill in the art will appreciate that one or more features of the device and method aspects of the present invention are optional. For example, a device may be made within the scope of the invention without a curve portion of the distal region. Further a pressure measuring mechanism for positioning the device is optional. In other instances, the ECG monitoring feature is optional as pressure may be used for positioning the device.

Although the above description relates to specific embodiments as presently contemplated by the inventors, it is understood that the invention in its broad aspect includes mechanical and functional equivalents of the elements described herein.

The invention will now be described by reference to the following statements. These statements are numbered but are not claims. These statements relate to the claims of the parent application and are included for completeness to preserve all subject matter.
1. A surgical device for cutting material and monitoring ECG comprising:
   an elongate member having a distal region and a proximal region;
   an energy delivery device associated with the elongate member at the distal region for delivering cutting energy to the material, said energy delivery device adapted for connection to an energy source; and
   an ECG monitoring device associated with the distal region for monitoring ECG, said ECG monitoring mechanism adapted for connection to an ECG recording device.
2. The device of embodiment 1 wherein the energy delivery device is configured to deliver at least one form of cutting energy selected from a group consisting of: electrical energy; microwave energy; ultrasound energy; and laser energy.
3. The device of embodiment 1 wherein the energy delivery device is configured to deliver electrical energy comprising electrical current having a frequency within the radio frequency range.
4. The device of embodiment 1 wherein the material comprises cellular tissue and wherein the energy delivery device is operable to deliver sufficient energy to the tissue to result in a rapid increase in the intracellular temperature causing vaporization of intracellular water and subsequent cell lysis.
5. The device of embodiment 1 wherein the ECG monitoring mechanism comprises at least one active electrode about the distal region adapted for connection to an ECG recording device.
6. The device of embodiment 5 wherein the distal region comprises two or more electrodes about the distal region adapted for connection to an ECG recording device; and wherein the electrodes are configured in an arrangement where at least one of the electrodes is active and at least one is a reference electrode.
7. The device of embodiment 5 wherein the surgical device is insertable into a patient's vasculature using at least one of a guiding sheath and a dilator; and wherein at least one reference electrode is located on a distal region of at least one of the sheath and the dilator.
8. The device of embodiment 1 wherein the energy delivery device and the ECG monitoring device comprise one and the same active electrode.
9. The device of embodiment 1 wherein a pressure sensing mechanism is associated with the distal region for monitoring pressure about the distal region.
10. The device of embodiment 9 wherein the transmitting lumen extending between the proximal and distal regions, said lumen at the proximal region being adapted for fluid communication with a pressure transducer that provides a signal which varies as a function of pressure and adapted at the distal region for fluid communication with an environment about said distal region.
11. The device of embodiment 10 wherein the lumen is adapted for injecting a fluid through at least one opening formed by the distal region.
12. The device of embodiment 11 wherein the distal region forms a plurality of openings for injecting a fluid and wherein some of the openings are located distally and some of the multiple openings are located proximally with respect to each other and wherein the some of the openings located distally are larger than the some of the openings located proximally.
13. The device of embodiment 9 wherein the pressure sensing mechanism comprises a pressure transducer on-board the elongate member, said transducer being adapted for communication with a pressure monitoring system.
14. The device of embodiment 1 wherein the distal region comprises a portion having a curved shape to direct the energy delivery device in a desired direction.
15. The device of embodiment 14 wherein the portion having a curved shape defines a radial arc.
16. The device of embodiment 14 wherein the distal region comprises a straight portion about 1 cm in length located distally of the portion having a curved shaped.
17. The device of embodiment 14 wherein the proximal region comprises a marking indicative of the orientation of the portion having a curved shape.
18. An electrosurgical device comprising:
   an elongate member having a distal region and a proximal region, said distal region insertable within and along a lumen within a body of a patient
      and maneuverable therethrough to a desired location where the device is operated to cut material and monitor ECG at the desired location;
   at least one electrode associated with the distal region for cutting tissue, said at least one electrode adapted for coupling to an electrical power source; and
   an ECG monitoring mechanism associated with the distal region for monitoring ECG at the desired location within the body, said mechanism adapted for coupling to an ECG recording device.
19. The device of embodiment 18 wherein the ECG monitoring mechanism is configured to minimize a portion of the elongate member that is necessary to be located at the desired location to monitor ECG.
20. The device of embodiment 18 wherein the at least one electrode defines a functional tip comprising a conductive and radiopaque material at said distal region.
21. The device of embodiment 20 wherein the electrical power source provides a highfrequency electrical power to said functional tip in a high impedance range.
22. The device of embodiment 18 wherein the proximal region is adapted to releasably couple said 10 electrode to said electrical power source.
23. The device of embodiment 18 wherein the proximal region is adapted to releasably couple said ECG monitoring mechanism to said ECG recording device.
24. A surgical device comprising:
   means for cutting material at a desired location in a body of a patient; and
   means for determining a position of the device responsive to ECG within the heart.
25. The device of embodiment 24 comprising a flexible elongate member having a proximal region and a distal region, said distal region adapted for insertion within and along a lumen within the body and maneuverable therethrough to the desired location; and wherein said means for determining a position of the device is operable to determine the position of the distal region.
26. A surgical device for cutting material and monitoring pressure comprising:
   an elongate member having a distal region and a proximal region;
   an energy delivery device associated with the elongate member at the distal region for delivering cutting energy to the material, said energy delivery device adapted for connection to an energy source; and
   a pressure sensing mechanism associated with the distal region for monitoring pressure about the distal region.
27. The device of embodiment 26 wherein the energy delivery device is configured to deliver at least one form of cutting energy selected from a group consisting of: electrical energy; microwave energy; ultrasound energy; and laser energy.
28. The device of embodiment 26 wherein the energy delivery device is configured to deliver electrical energy comprising electrical current having a frequency within the radio frequency range.
29. The device of embodiment 26 wherein the material comprises cellular tissue and wherein the energy delivery device is operable to deliver sufficient energy to the tissue to result in a rapid increase in the intracellular temperature causing vaporization of intracellular water and subsequent cell lysis.
30. The device of embodiment 26 wherein the pressure sensing mechanism comprises a pressure transmitting lumen extending between the proximal and distal 'regions, said lumen at the proximal region being adapted for fluid communication with a pressure transducer that provides a signal which varies as a function of pressure and adapted at the distal region for fluid communication with an environment about said distal region.
31. The device of embodiment 30 wherein the distal region comprises at least one opening to the environment and wherein the lumen is in fluid communication with the at least one opening.
32. The device of embodiment 31 wherein the lumen is adapted for injecting a fluid through the at least one opening.
33. The device of embodiment 32 wherein the at least one opening of the distal region comprises multiple openings to the environment and wherein some of the multiple openings are located distally and some of the multiple openings are located proximally with respect to each other and wherein the some of the openings located distally are larger than the some of the openings located proximally.
34. The device of embodiment 26 wherein the pressure sensing mechanism comprises a pressure transducer on-board the elongate member, said transducer being adapted for communication with a pressure monitoring system.
35. The device of embodiment including an ECG monitoring device associated with the distal region for monitoring ECG about the distal region.
36. The device of embodiment 35 wherein said ECG monitoring device is adapted for connection to an ECG recording device.
37. The device of embodiment 35 wherein the ECG monitoring mechanism comprises at least one active electrode about the distal region.
38. The device of embodiment 26 wherein the distal region comprises a portion having a curved shape to direct the energy delivery device in a desired direction.
39. The device of embodiment 26 wherein, when the energy delivery device advances through the material, the distal region adopts a curved shape to direct the energy delivery device in a desired direction.
40. The device of embodiment 38 wherein the portion having a curved shape defines a radial arc.
41. The device of embodiment 38 wherein the proximal region comprises a marking indicative of the orientation of the portion having a curved shape.
42. An electrosurgical device comprising:
   a elongate member having a distal region and a proximal region, said distal region insertable within and along a lumen within a body of a patient and maneuverable therethrough to a desired location where the device is operated to cut material and monitor pressure at the desired location;
   at least one electrode associated with the distal region for cutting tissue, said at least one electrode adapted for coupling to an electrical power source; and
   a pressure sensing mechanism associated with the distal region for sensing pressure at the desired location within the body, said mechanism adapted for coupling to a pressure monitoring system.
43. The device of embodiment 42 wherein the pressure sensing mechanism is configured to minimize a portion of the elongate member that is necessary to be located at the desired location to monitor pressure.
44. The device of embodiment 42 wherein the pressure sensing mechanism comprises a pressure transmitting lumen defined within the elongate member extending from the proximal region to and through at least one opening defined in the distal region.
45. The device of embodiment 44 wherein said proximal region is adapted for coupling said pressure transmitting lumen to a pressure transducer associated with the pressure monitoring system.
46. The device of embodiment 44 wherein the pressure transmitting lumen is adapted for at least one of injecting a fluid to or removing a fluid from said body.
47. The device of embodiment 44 wherein the at least one electrode is coupled to said energy source by a coupling means extending through said pressure transmitting lumen.
48. The device of embodiment 42 wherein said pressure sensing mechanism comprises an on-board pressure transducer adapted for communicating a transduced pressure signal representative of pressure about the distal region to said pressure monitoring system.
49. The device of embodiment 42 wherein the at least one electrode defines a functional tip comprising a conductive and radiopaque material at said distal region.
50. The device of embodiment 49 wherein the electrical power source is capable of providing a high-frequency electrical power to said functional tip in a high impedance range.
51. The device of embodiment 42 wherein the proximal region is adapted to releasably couple said pressure sensing mechanism to said pressure monitoring system.
52. The device of embodiment 42 wherein the proximal region is adapted to releasably couple said electrode to said electrical power source.
53. A surgical device comprising:
   means for cutting material at a desired location in a body of a patient; and
   means for determining a position of the device responsive to pressure within the body.
54. The device of embodiment 53 comprising a flexible elongate member having a proximal region and a distal region, said distal region adapted for insertion within and along a lumen within the body and maneuverable therethrough to the desired location; and wherein said means for determining a position of the device is operable to determine the position of the distal region:
55. The device of embodiment 53 wherein the means for determining a position of the device comprises a pressure transducer for providing a signal representative of pressure to a pressure monitoring system.
56. The device of embodiment 55 wherein the means for determining a position of the device comprises a pressure transmitting lumen defined by the device for coupling to the pressure transducer.
57. The device of embodiment 56 comprising a means for injecting fluid to and removing fluid from the body.
58. A device for creating a perforation in material within a patient comprising:
   an elongate member having a proximal region and a distal region capable of adopting a curved shape; and
   a functional tip at the distal region for delivering energy to create the perforation in the material;
   wherein when the functional tip advances through the material, the distal region adopts a curved shape to direct the functional tip in a desired direction.
59. The device of embodiment 58 wherein the material comprises a body tissue.
60. The device of embodiment 58 wherein the distal region comprises a portion having a curved shape.
61. The device of embodiment 59 wherein the portion having a curved shape defines a radial arc.
62. The device of embodiment 61 wherein the distal region comprises a straight portion of about 1 cm in length located distally of the potion having a curved shape.
63. The device of embodiment 58 wherein the proximal region comprises a marking indicative of the orientation of the distal region.
64. A device for creating a perforation in a heart septum comprising:
   an elongate member having a proximal region and a distal region, the elongate member insertable along a lumen to position the distal region to the heart septum, the distal region comprising a portion having a curved shape when unconstrained by the lumen; and
   a functional tip at the distal region for delivering energy to create the perforation in the septum;
   wherein when the distal region advances through the septum unconstrained by the lumen, the distal region adopts a curved shape to direct the functional tip in a desired direction.
65. The device of embodiment 64 wherein the portion having a curved shape defines a radial arc.
66. The device of embodiment 64 wherein the proximal region comprises a marking indicative of an orientation of the distal region.
67. The device of embodiment 64 wherein the energy delivery device is configured to deliver at least one form of cutting energy selected from a group consisting of: electrical energy; microwave energy; ultrasound energy; and laser energy.
68. The device of embodiment 64 wherein the energy delivery device is configured to deliver electrical energy comprising electrical current having a frequency within the radio frequency range.
69. The device of embodiment 64 wherein the heart functional tip delivers sufficient energy to the tissue to result in cell lysis.
70. The device of embodiment 64 comprising a pressure sensing mechanism associated with the distal region for monitoring pressure about the distal region.
71. The device of embodiment 70 wherein the pressure sensing mechanism comprises a pressure transmitting lumen extending between the proximaland distal regions, the lumen adapted at the proximal region for fluid communication with a pressure transducer and adapted at the distal region for fluid communication with an environment about the distal region.
72. The device of embodiment 71 wherein the distal region defines at least one opening to the 15 environment and wherein the lumen is in fluid communication with the at least one opening.
73. The device of embodiment 70 wherein the pressure sensing mechanism comprises a pressure transducer on-board the distal region and adapted for communication with a pressure monitoring system.
74. The device of embodiment 64 wherein the distal region comprises a straight portion having a length of about 1 cm located distally of the portion having a curved shape.
75. The device of embodiment 74 wherein the portion having a curved shape defines a length of about 6cm and wherein the curved shape extends about 270 degrees of the circumference of a circle.
76. The device of embodiment 64 comprising at least one depth marking.
77. The device of embodiment 64 comprising an ECG monitoring device associated with the distal region for monitoring ECG about the distal region.
78. The device of embodiment 77 wherein the ECG monitoring mechanism comprises at least one active electrode about the distal region adapted for connection to an ECG recording device.
79. An electrosurgical device comprising:
   an elongate member having a proximal region and a distal region comprising a functional tip, the distal region shaped such that the functional tip is directed towards a desired location after perforating an interatrial septum;
   at least one electrode associated with the functional tip for cutting tissue, the at least one electrode adapted for coupling to an electrical energy source; and
   a pressure sensing mechanism associated with the distal region for sensing pressure at a desired location within a patient, the mechanism adapted for coupling to a pressure monitoring system.
80. The device of embodiment 79 wherein the pressure sensing mechanism comprises a pressure transmitting lumen in communication with at least one opening about the distal region for at least one of injecting a fluid to or removing a fluid from the patient.
81. The device of embodiment 80 wherein the at least one electrode is coupled to the electrical energy source by a coupling means extending through the pressure transmitting lumen.
82. The device of embodiment 79 wherein the pressure sensing mechanism comprises an on-board pressure transducer adapted for communicating a transduced pressure signal representative of pressure about the distal region to the pressure monitoring system.
83. The device of embodiment 79 comprising an ECG monitoring device associated with the distal region for monitoring ECG about the distal region.
84. A device for creating a perforation in a heart septum comprising:
   an elongate member having a distal region and a proximal region;
   a functional tip at the distal region for delivering energy to create the perforation in the septum; and
   a control associated with the distal region and operable from the proximal region, wherein the control is operable to modify a shape of the distal region to direct the functional tip in a desired direction.
85. A method of surgery comprising the steps of:
   (i) introducing a surgical device into a heart of a patient, the surgical device comprising an elongate member having a distal region and a proximal region, an energy delivery device proximate to the distal region capable of cutting material and an ECG monitoring mechanism for determining ECG in the heart proximate to the distal region;
   (ii) positioning the energy delivery device to a first desired location in the heart adjacent material to be cut;
   (iii) delivering energy using the energy delivery device to cut said material; and
   (iv) monitoring ECG in the heart using the ECG monitoring mechanism in order to determine the position of the surgical device at least one of before and after step (iii).
86. The method of embodiment 85 further comprising a step of:
   (v) moving the device to a second location.
87. The method of embodiment 86 further comprising a step of:
   (vi) monitoring ECG using the ECG monitoring mechanism at the second location.
88. The method of embodiment 85 wherein step (i) comprises introducing the device into the patient's vasculature.
89. The method of embodiment 88 wherein the step of introducing the device into the patient's vasculature comprises inserting the device into a dilator and a guiding sheath positioned in the patient's vasculature.
90. The method of embodiment 89 comprising a step of:
   (v) moving the dilator and the sheath to a second location together over the surgical device.
91. The method of embodiment 89 comprising a step of:
   (v) moving the dilator, sheath and surgical device all together to a second location.
92. The method of embodiment 85 wherein the material is tissue located on an atrial septum of the heart.
93. The method of embodiment 87 wherein the ECG monitored at the second location is the ECG in the left atrium.
94. The method of embodiment 85 wherein step (ii) comprises dragging the surgical device about a surface of the heart while simultaneously monitoring ECG to determine the first desired location.
95. The method of embodiment 94 wherein the first desired location is determined in response to the observation of a distinctive change in the ECG signal.
96. The method of embodiment 95 wherein the ECG signal at the first desired location is damped in comparison with the ECG signal monitored otherwise on the surface of the heart as the surgical device is located about the first desired location.
97. The method of embodiment 95 wherein the first desired location is a fossa ovalis of the heart.
98. A method of cutting tissue at a desired location in a body of a patient comprising the steps of:
   inserting a surgical device into the body, said surgical device comprising means for cutting material and means for determining a position of the device responsive to ECG within the heart; and
   positioning said surgical device at the desired location in response to the means for determining a position of the device.
99. The method of embodiment 98 comprising the step of:
   cutting material at the desired location.
100. The method of embodiment 99 comprising the step of:
   moving said device in the body in response to said means for determining a position of the device.
101. The method of embodiment 100 comprising repositioning said device for re-cutting in response to 10 said means for determining a position of the device.
102. A method of surgery comprising the steps of:
   (i) introducing a surgical device into a body of a patient, the surgical device comprising an elongate member having a distal region and a proximal region, an energy delivery device proximate to the distal region capable of cutting material and a pressure sensing mechanism for determining pressure in the body proximate to the distal region;
   (ii) positioning the energy delivery device to a first desired location in the patient's body adjacent material to be cut;
   (iii) delivering energy using the energy delivery device to cut said material; and
   (iv) measuring pressure in the body using the pressure sensing mechanism in order to determine the position of the surgical device at least one of before and after step (iii).
103. The method of embodiment 102 wherein step (ii) comprises staining a region of tissue in the first desired location in the patient's body.
104. The method of embodiment 102 further comprising a step of:
   (v) moving the device to a second desired location.
105. The method of embodiment 104 wherein the surgical device comprises at least one depth marking and at least one radiopaque marker and wherein step (v) comprises monitoring at least one of said depth markings and at least one of said radiopaque markers.
106. The method of embodiment 104 further comprising a step of:
   (vi) measuring pressure using the pressure sensing mechanism at the second location.
107. The method of embodiment 106 wherein the surgical device comprises at least one depth marking and at least one radiopaque marker and wherein step (vi) is performed after confirming the position of the pressure sensing mechanism at the second location using at least one of said depth markings and said radiopaque markers.
108. The method of embodiment 102 wherein step (i) comprises introducing the device into the patient's vasculature.
109. The method of embodiment 108 wherein the step of introducing the device into the patient's vasculature comprises inserting the device into a dilator and a guiding sheath positioned in the patient's vasculature.
110. The method of embodiment 109 wherein the device and at least one of the dilator and sheath comprise a radiopaque marking and wherein step (ii) comprises aligning the radiopaque markings to aid in positioning the device.
111. The method of embodiment 110 comprising a step of:
   (v) moving the dilator and the sheath to a second location together over the surgical device.
112. The method as claimed in item 110 comprising a step of:
   (v) moving the dilator, sheath and surgical device all together to a second location.
113. The method of embodiment 102 wherein the material is tissue located on an atrial septum of a heart.
114. The method of embodiment 103 wherein the region of tissue to be stained is a fossa ovalis of a heart.
115. The method of embodiment 106 wherein the pressure measured at the second location is the blood pressure in the left atrium.
116. A method of cutting tissue at a desired location in a body of a patient comprising the steps of:
   inserting a surgical device into the 'body, said surgical device comprising means for' cutting material and means for determining a position of the device responsive to pressure within the body; and
   positioning said surgical device at the desired location in response to the means for determining a position of the device.
117. The method of embodiment 116 comprising the step of:
   cutting material at the desired location.
118. The method of embodiment 117 comprising the step of:
   moving said device in the body in response to said means for determining a position of the device.
119. The method as claimed in item 118 comprising repositioning said device for re-cutting in response to said means for determining a position of the device.
120. A method of creating a perforation in a heart septum comprising:
   applying a form of energy to a perforation device positioned at a desired location of a heart septum to create a perforation at the desired location, wherein the perforation device comprises an elongate member having a proximal region and a distal region capable of adopting a curved shape; and
   while moving a distal tip of the device through the septum, directing the distal tip in a desired direction.
121. The method of embodiment 120 wherein the perforation device comprises a control associated with the distal region and operable from the proximal region, wherein the control is operable to modify a shape of the distal region to direct the distal tip in a desired direction in relation to cardiac structures; and wherein the method comprises operating the control.
122. The method of embodiment 120 wherein the method comprises manipulating the distal region to adopt the curved shape.
123. The method of embodiment 120 wherein the distal tip is directed away from cardiac structures in order to decrease risk of unwanted injury.
124. The method of embodiment 120 wherein the perforation device comprises a pressure sensing mechanism for sensing pressure at the distal tip and wherein the method comprises monitoring the pressure to indicate a location of the distal tip.
125. The method of embodiment 120 wherein the perforation device comprises an orientation indicator for determining a direction of the distal tip and wherein the method comprises monitoring the orientation indicator to move the distal tip through the septum in a desired direction.
126. A surgical device for perforating material and monitoring pressure comprising:
   an elongate member having a distal region with a first outer diameter and a proximal region with a second outer diameter larger than said first outer diameter such that dilation of a perforation is limited while said distal region is advanced through said perforation, wherein said second outer diameter tapers down to said first outer diameter so as to provide a smooth transition between said first outer diameter and said second outer diameter;
   an energy delivery device associated with said elongate member at said distal region, said energy delivery device adapted for connection to an energy source and operable to effectively perforate said material by delivering energy; and a pressure sensing mechanism associated with said distal region for monitoring pressure about said distal region;
   wherein said pressure sensing mechanism comprises a pressure transmitting lumen extending between said proximal and distal regions, the lumen at said proximal region being adapted for fluid communication with a pressure transducer that provides a signal which varies as a function of pressure and adapted at said distal region for fluid communication with an environment about said distal region.
127. The device of embodiment 127, wherein said energy is at least one form of energy selected from a group consisting of: electrical current; microwave; ultrasound; and laser.
128. The device of embodiment 128 wherein said electrical current has a frequency within the radio frequency range.
129. The device of any preceding embodiment, wherein said material comprises cellular tissue and wherein said energy delivery device is operable to deliver sufficient energy to the tissue to result in a rapid increase in the intracellular temperature causing vaporization of intracellular water and subsequent cell lysis.
130. The device of any preceding embodiment, wherein said distal region comprises at least one opening to the environment and wherein said lumen is in fluid 10 communication with said at least one opening.
131. The device of any preceding embodiment, wherein said energy delivery device comprises a functional tip with at least one active electrode.
132. The device of embodiment 131, said functional tip having two or more electrodes.
133. The device of embodiment 132, wherein the electrodes are configured in an arrangement where at least one of said electrodes is active and at least one is a return electrode.
134. The device of embodiment 131, wherein said at least one electrode comprises an active electrode with an outer diameter of substantially 0.04 cm.
135. The device, of any proceeding embodiment, wherein said pressure transmitting lumen is adapted for at least one of injecting a fluid to or removing a fluid from said body.
136. The device of embodiment 131, wherein said at least one active electrode is coupled to said energy source by a coupling means extending through said pressure transmitting lumen.
137. The device of embodiment 131, wherein said at least one active electrode defines a functional tip comprising a conductive and radiopaque material at said distal region.
138. The device of any preceding embodiment, wherein said energy source is capable of providing high-frequency electrical power in a high impedance range.
139. A system for perforating material and monitoring pressure comprising:
   a device comprising:
      an elongate member having a distal region with a first outer diameter and a proximal region with a second outer diameter larger than said first outer diameter such that dilation of a perforation is limited while said distal region is advanced through said perforation, wherein said second outer diameter tapers down to said first outer diameter so as to provide a smooth transition between said first outer diameter and said second outer diameter;
      an energy delivery device associated with said elongate member at said distal region; and
      a pressure sensing mechanism associated with said distal region for monitoring pressure about said distal region;
      wherein said pressure sensing mechanism comprises a pressure transmitting lumen extending between said proximal and distal regions, the lumen at said proximal region being adapted for fluid communication with a pressure transducer that provides a signal which varies as a function of pressure and adapted at said distal region for fluid communication with an environment about said distal region; and
      an energy source capable of providing high-frequency electrical power in a high impedance range;
      said energy delivery device adapted for connection to said energy source and operable to effectively perforate said material by delivering energy received from said energy source.

## Claims

1. A device for creating a controlled transseptal perforation, the device comprising:
- a tubular elongate member defining a proximal region and a distal region;
- at least one electrode associated with the distal region, the at least one electrode being capable of creating the controlled transseptal perforation in body tissue through the application of radiofrequency energy; and
- a means for determining a position of the device inside a patient's body.

2. The device of claim 1, wherein the means for determining the position comprises:
- a lumen extending from the proximal region to the distal region of the elongate member; and
- at least one opening at the distal region in fluid communication with the lumen and with an environment surrounding the distal region.

3. The device of any one of claims 1 or 2, wherein a curve portion of the elongate member is curved.

4. The device of claim 3, wherein the curve portion of the elongate member is constructed from an elastic material whereby in use the curve portion conforms to an internal lumen of a dilator when inserted therein and wherein the curve portion takes on a curved shape after being advanced out of the dilator.

5. The device of claim 2, further comprising external tubing coupled to the proximal region for connection to a pressure transducer.

6. The device of claim 1, wherein the means for determining the position comprises a pressure transducer associated with the distal region.

7. The device of claim 4, wherein the distal region is atraumatic.

8. The device of claim 4, wherein the at least one electrode is configured in a partially-annular shape.

9. The device of claim 3, wherein an outer diameter of the elongate member tapers to the distal region.

10. The device of any one of claims 4 to 8, wherein the elongate member has an outer diameter no larger than 0.050 inches (1.282 mm).

11. The device of any one of claims 4 to 9, further comprising a hub at the proximal region, the hub including a connector cable terminating in a connector.

12. The device of claim 3, further comprising a hub at the proximal region, the hub including an orientation indicator for indicating a direction of the curve portion.
